# EUROPEAN PATENT SPECIFICATION

(11) **EP 2 773 201 B1**
(45) Date of publication and mention of the grant of the patent: **06.12.2017**
(21) Application number: 12846028.4
(22) Date of filing: 31.10.2012
(51) Int. Cl.: A01N 37/18, A61K 9/00, A61K 47/18, A61K 47/44, A61K 33/18, A61K 9/10

(54) **IMPROVED COMPOSITIONS COMPRISING POVIDONE-IODINE COMPLEX**
VERBESSERTE ZUSAMMENSETZUNGEN BEINHALTEND POVIDONE-IOD KOMPLEX
COMPOSITIONS AMÉLIORÉES COMPRENANT DE LA POVIDONE IODÉE

(30) Priority: 31.10.2011 US 201161553503 P
(43) Date of publication of application: 10.09.2014
(73) Proprietor: Microdermis Corporation, Grand Prairie, TX 75051 (US)
(72) Inventor: LENTINI, Peter, Tarrytown, NY 10591 (US)
(74) Representative: Schiweck, Weinzierl & Koch Patentanwälte Partnerschaft mbB
(86) International application number: PCT/US2012/062643
(87) International publication number: WO 2013/066909

(56) References cited:
- WO-A2-2011/140310
- US-A- 5 716 611
- US-A1- 2010 197 795
- US-A1- 2010 197 795
- US-A1- 2010 203 005
- US-B1- 6 436 379
- Anonymous: "Povidone-Iodine: Martindale: The Complete Drug Reference", , 20 May 2004 (2004-05-20), XP55182455, Retrieved from the Internet: URL:https://www.medicinescomplete.com/mc/m artindale/current/4580-y.htm?q=povidone%20 iodine&t=search&ss=text&p=1#_hit [retrieved on 2015-04-13]

## Description

### Background of the Invention

Povidone-iodine compositions (i.e., solutions, scrubs and other topical preparations) commonly contain a combination of a low pH and aggressive surfactants. This combination can lead to dry, tight, cracked and/or uncomfortable skin of repeated users. Some povidone-iodine compositions have been prepared to include glycerine and other humectants in order to minimize skin drying. These compositions can be limited in that they only provide humectancy, which is short-lived moisturization, and eventually will lead to dry skin on repeated use. This in turn can lead to reduced compliance among users (e.g., those in a hospital environment) and greater risk of infections. Additionally, these compounds are not necessarily stable to iodine, in that the humectants have medium to high iodine numbers. Thus the resulting povidone-iodine compositions do not demonstrate long term stability with respect to iodine. Patent document US 5716611 discloses formulations comprising povidone iodine.

### Summary of the Invention

The present invention encompasses the insight that use of particular emollients, and in particular of low iodine reactivity emollients, permits improved povidone-iodine compositions. In one aspect, the present invention encompasses identification of the source of a problem with certain prior art povidone-iodine compositions, i.e., that such compositions utilize one or more components that reacts with iodine such that one or both of the iodine and the reactive component is depleted or disrupted in the composition. The present invention therefore provides compositions and strategies that protect particular components of and/or preserve one or more desirable features of certain povidone-iodine compositions.

In various aspects, the present invention provides a composition including: 4 weight percent to 12 weight percent of povidone-iodine complex, 0.1 weight percent to 2 weight percent of a tertiary amide, tertiary amide salt, and/or tertiary amide hydrate, 0.5 weight percent to 2 weight percent of low iodine reactive emollient, wherein the low iodine reactive emollient is selected from the group consisting of jojoba oil, hydrogenated jojoba esters, hydrogenated polybutenes, isododecane, glyceryl tribehenate, hydrogentated castor oil, palm oil, derivatives thereof and combinations thereof; at least one surfactant; and water; wherein the composition has a pH ranging from 2.0 to 3.5.

In some embodiments, the weight percent of the povidone-iodine complex is between about 5 and about 10. In some embodiments, the weight percent of the povidone-iodine complex is between about 6 and about 9.

In some embodiments, the low iodine reactive emollient has an iodine number of less than about 80. In some embodiments, the low iodine reactive emollient has an iodine number of less than about 25. In some embodiments, the low iodine reactive emollient has an iodine number of less than about 10. In some embodiments, the low iodine reactive emollient has an iodine number of less than about 5. The low iodine reactive emollient is selected from the groups consisting of jojoba oil, hydrogenated jojoba esters, hydrogenated polybutenes, isododecane, glyceryl tribehenate, hydrogentated castor oil, palm oil, derivatives thereof and combinations thereof. In some embodiments, the low iodine reactive emollient includes hydrogenated jojoba esters. In some embodiments, the low iodine reactive emollient is jojoba oil.

In some embodiments, the surfactant is selected from the group consisting of a polyethylene glycol of 4-(1,1,13 3-tetramethybutyl)-phenyl, glyceryl stearate, PEG-100 stearate, laureth-12, Non-oxynol-9 or combinations thereof.

In some embodiments, the tertiary amide, tertiary amide hydrate, tertiary amide salt has the formula: 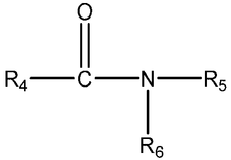
wherein R₄ is a saturated fatty group containing 11-60 carbon atoms;
R₅ and R₆ are independently aryl, aryl lower alkyl, a saturated fatty group containing 11-29 carbon atoms, or R₇; and
R₇ is

   R₁-Ar-O-R₂-O-R₃
wherein R₂ and R₃ are lower alkyl groups containing 1-6 carbon atoms and R₁ is a lower alkyl group and Ar is aryl.

In some embodiments, R₄ is a saturated fatty group containing 15-46 carbon atoms. In some embodiments, Ar is a phenyl group. In some embodiments, R₅ is a benzyl group. In some embodiments, the tertiary amide is benzethonium stearamide or benzalkonium stearamide. In some embodiments, the composition is in the form of a paste, cream or ointment.

In various aspects, the present invention provides a composition including: about 4 weight percent to about 12 weight percent of povidone-iodine complex, about 0.5 weight percent to about 2 weight percent of an emollient with low iodine reactivity, at least one surfactant; and water, wherein the composition has a pH ranging from about 2.0 to about 3.5.

In some embodiments, the weight percent of the povidone-iodine complex is between about 5 and about 10. In some embodiments, the weight percent of the povidone-iodine complex is between about 6 and about 9.

In some embodiments, the low iodine reactive emollient has an iodine number of less than about 80. In some embodiments, the low iodine reactive emollient has an iodine number of less than about 25. In some embodiments, the low iodine reactive emollient has an iodine number of less than about 10. In some embodiments, the low iodine reactive emollient has an iodine number of less than about 5. The low iodine reactive emollient is selected from the groups consisting of jojoba oil, hydrogenated jojoba esters, hydrogenated polybutenes, isododecane, glyceryl tribehenate, hydrogentated castor oil, palm oil, derivatives thereof and combinations thereof. In some embodiments, the low iodine reactive emollient includes hydrogenated jojoba esters. In some embodiments, the low iodine reactive emollient is jojoba oil.

In some embodiments, the surfactant is selected from the group consisting of a polyethylene glycol of 4-(1,1,13 3-tetramethybutyl)-phenyl, glyceryl stearate, PEG-100 stearate, laureth-12, Non-oxynol-9 or combinations thereof.

In some embodiments, the tertiary amide, tertiary amide hydrate, tertiary amide salt has the formula: 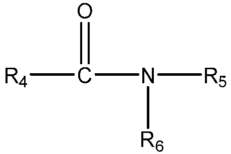
wherein R₄ is a saturated fatty group containing 11-60 carbon atoms;
R₅ and R₆ are independently aryl, aryl lower alkyl, a saturated fatty group containing 11-29 carbon atoms, or R₇; and
R₇ is

   R₁-Ar-O-R₂-O-R₃
wherein R₂ and R₃ are lower alkyl groups containing 1-6 carbon atoms and R₁ is a lower alkyl group and Ar is aryl.

In some embodiments, R₄ is a saturated fatty group containing 15-46 carbon atoms. In some embodiments, Ar is a phenyl group. In some embodiments, R₅ is a benzyl group. In some embodiments, the tertiary amide is benzethonium stearamide or benzalkonium stearamide. In some embodiments, the composition is in the form of a paste, cream or ointment.

In various aspects the present invention presents a composition formed by the combination of: povidone-iodine, low iodine reactive emollient; and the combination of: a nitrogenous base, a C₁₂ - C₆₀ fatty acid; and a C₁₂ - C₂₁ dialkyl quaternary ammonium salt.

### Brief Description of the Drawings

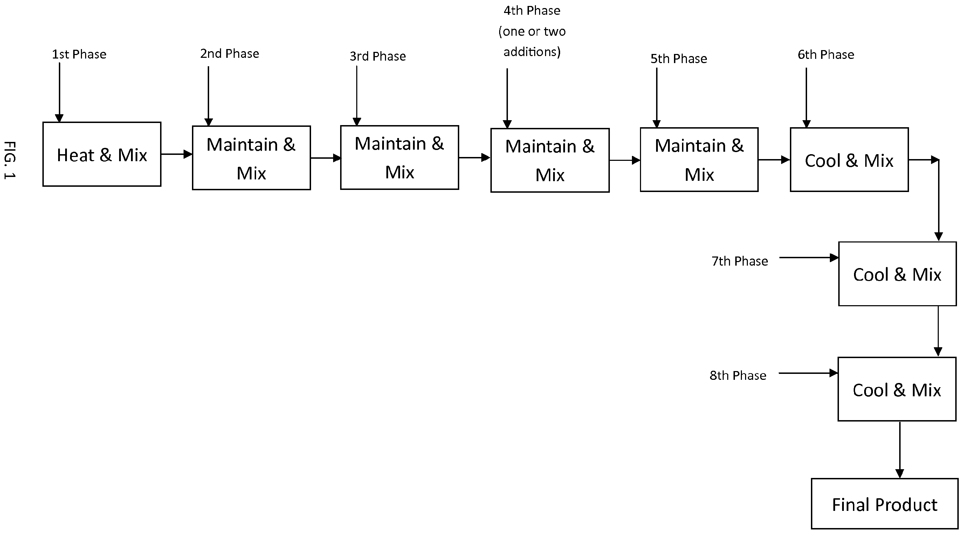

Fig. 1 depict and exemplary flow chart of the phase additions and operations involved in producing exemplary product. The method depicted in the flow chart of Fig. 1 is further described in the Examples below.

### Definitions

*Iodine number or iodine value,* as used herein, is the number of grams of iodine taken up by 100 grams of a compound (i.e., an oil, fat, wax). In practice, iodine number is measured by mixing a known quantity of iodine (e.g., iodine monochloride) in excess with a known weight of the testing compound (i.e., the oil, fat, wax), then determining the amount of unreacted iodine by titration. Thus, by subtraction the amount of iodine reacted with the test compound is determined. This number is normalized to a 100 gram sample of the test compound, giving the iodine number. See also, ASTM D5786-02 (2006) and/or DIN 53241. Iodine number is indicative of the degree of unsaturation of a compound. Saturated fats, oils and waxes do not tend to react, or are less reactive with iodine, as compared to unsaturated fats, oils and waxes. Unsaturated fats, oils and waxes, however, do react with iodine or are more strongly reactive with iodine, as the iodine reacts with double and triple bonds found in unsaturated compounds.

*Low iodine reactive compounds,* as used herein, refers to compounds with low iodine number (e.g., less than about 80, less than about 60, less than about 40, less than about 20, less than about 10, less than about 5, less than about 2 or about zero). In the context of oils, these are often referred to as "non-drying oils," described below. Exemplary oils in this category include, but are not limited to olive oil, coconut oil, peanut oil. Other fats and waxes with low iodine reactivity include beeswax and jojoba wax. Additionally, some synthetic waxes have low iodine reactivity including polyethylene and mineral wax.

*Intermediate iodine reactive compounds,* as used herein, refers to compounds with iodine numbers in the range of between about 80 and about 130 (e.g about 90, about 100, about 110, about 120 and ranges therebetween). And, in the context of oils, may also be referred to as "semi-drying oils." Exemplary semi-drying oils include, but are not limited to, corn oil, soybean oil, cottonseed oil. Other fats and waxes with intermediate iodine reactivity include shea butter, avocado butter and carnuba wax.

*High iodine reactive compounds,* as used herein, refers to compound with iodine number above about 130. And again, in the context of oil, these are referred to as "drying oils." In oils, the double and triple bonds, undergo auto-oxidation upon exposure to air. This results in hardening, or drying, of the oil as fatty acid chains become cross-linked. The oil is hardened by this process, as opposed to solvent evaporation. Thus, because high iodine number is indicative of a high number of double and/or triple bonds, high iodine number compounds will also undergo drying, and thus can be termed drying oils. Exemplary drying oils, include, but are not limited to tung oil, linseed oil, sesame oil, safflower oil, almond oil, rice bran oil or wax, hazelnut oil and argan oil.

### Detailed Description of Certain Embodiments of the Invention

### Povidone-iodine compositions

In some aspects, provided compositions provide topical compositions that have antiseptic effects. In some embodiments, provided compositions contain povidone-iodine as an antiseptic and a low iodine reactive emollient. Provided compositions as a whole exhibits long term stability and are effective antiseptics after 6, 12, 18 or 24 months of storage. Stability refers to the activity of available iodine (e.g. after a storage period the composition retains 90% of the available iodine, as compared to pre-storage composition). Stability can be measured according to USP methods for determining available iodine, known in the art. In some embodiments, compositions retain 90% of available iodine after storage for four months at 40 °C and 75% relative humidity. In some embodiments, compositions retain 90 % of available iodine after storage for two years at room temperature and ambient relative humidity. In some embodiments, provided compositions include at least one surfactant and/or have a pH of less than about 3.5.

In some aspects, present inventions encompass the recognition that while povidone-iodine is effective as an antimicrobial, many povidone-iodine compositions may have deleterious effects, in particular with respect to conditioning of a user's skin. Povidone-iodine compositions (i.e., solutions, scrubs and other topical preparations) commonly contain a combination of a low pH and aggressive surfactants. This combination can lead to dry, tight, cracked and/or uncomfortable skin of repeated users. Present inventions include a series of emollients that mitigate and minimize the effects and the potential for drying skin. Some povidone-iodine compositions have been prepared to include glycerine and other humectants in order to minimize skin drying. In some embodiments, present inventions encompass recognition that such approaches can be limited in that they only provide humectancy, which is short-lived moisturization, and eventually will lead to dry skin on repeated use. This in turn can lead to reduced compliance among users (e.g., those in a hospital environment) and greater risk of infections. Additionally, these compounds are not necessarily stable to iodine, in that the humectants have medium to high iodine numbers. Thus the resulting povidone-iodine compositions do not demonstrate long term stability with respect to iodine.

In some embodiments, compositions provide povidone-iodine compositions that further include a tertiary amide, a salt or a hydrate thereof, which are described in more detail below. Without wishing to be bound by any particular theory, the present invention proposes that tertiary amides serve as a film forming agent on the skin, during application. The present invention specifically proposes that tertiary amides may form a hydrate with a central positive charge. Rubbing a composition comprising such a central positive charge may distribute positive charges over the skin surface. Negative charge of the skin may attract such positive charges, creating the distribution over the skin. Furthermore, positively charge moieties may penetrate the stratum corneum to achieve an electrostatic balance, so that the fatty layer becomes mechanically attached to the stratum corneum, creating a barrier.

In a further aspect, provided inventions include methods of use of the above provided compositions, also described in more detail below. Briefly, methods of use remove microbes (e.g., bacteria) from the skin of a subject (or the surface of an object). To do so, an antiseptically effective amount of the described composition is applied to an area of the skin, thoroughly rubbed into and on the skin to substantially cover the area of the skin to be disinfected. The method can be repeated as necessary or at regular intervals (e.g., every 1, 2, 3, 4, or 6 hours.). For application to objects, as opposed to subjects, an antiseptically effect amount of the composition is applied to the surface of the object to substantially cover the surface to be disinfected.

### Identification of the Source of a Problem

The present invention encompasses the recognition that use of emollients or other components that react with iodine can disrupt or destroy one or more components or features of povidone-iodine compositions. For example, the present invention provides the insight that low iodine reactive emollients will not only improve the efficacy of the antiseptic compositions, but can also reduce the drying effect on the user's skin. Furthermore, the stability of the compositions can be improved with low iodine reactive emollients, in turn improving shelf life of the compositions. Because emollients are selected to provide occlusivity, condition, moisture control and skin comfort in topical compositions, their influence extents not only to the stability and overall efficacy of the composition, but also to the efficacy and efficiency among users.

The present invention identifies various problems with existing povidone-iodine compositions, and also provides solutions to such identified problems. For example, the present inventions recognize that current povidone-iodine compositions result in dry, uncomfortable skin leading to patient/use discomfort, care-fiver hand dryness and damage on repeated use. Eventual consequence include non-compliance with hand-hygiene protocols in institutional settings. The instant inventions address these issues by providing an antiseptic compositions based on povidone-iodine, with low-iodine reactive emollients, providing both antiseptic properties and skin conditioning/emolliency and moisturizing, without compromising the efficacy of the antiseptic properties of the iodine and including improved stability of the compositions.

### Provided compositions

### Povidone-Iodine

Provided compositions contain povidone-iodine. Povidone-iodine is a chemically stable complex of polyvinylpyrollidone and elemental iodine. It is commercially available in forms with about 8 % to about 12% of complexed (or available) iodine. Iodine is a well-known disinfectant, with broad spectrum efficacy against bacteria (without developing resistance by the bacteria) as well as yeasts, molds, fungi and viruses. Iodine by itself (i.e., in solution in water or alcohol) does have drawbacks including staining, irritation at application and toxicity. These are overcome by complexing the iodine with a solublizing agent, producing an iodophor, such as povidone-iodine. Complexes (e.g. povidone-iodine) are water soluble and release free iodine when in solution. Povidone-iodine, in particular, is more stable in solution that other forms of iodine (e.g, Lugol's solution or tincture of iodine). Furthermore, the release rate of iodine from povidone-iodine is slow, which reduces toxicity and potential for irritation.

Though the iodine in povidone-iodine is complexed and stable by itself, it may still react with other compounds in a mixture. In particular reactions with carbon-carbon double bonds found in many topical compositions (e.g., in emollients) can consume the iodine, reducing the antiseptic efficacy of the composition, but also reducing the long term stability of the composition.

Povidone iodine complex, containing between about 8 percent and about 12 percent complexed iodine, can be between 1 weigh percent and about 40 weight percent of the final composition. In some embodiments, povidone-iodine is between about 3 weight percent and about 20 weight percent of the final composition, or between about 5 weight percent and about 10 weight percent.

### Tertiary Amide

In some embodiments, provided compositions include one or more tertiary amide, and/or components that can or do react to generate a tertiary amide. In some embodiments, an included tertiary amide is in the form of a salt or hydrate. In some embodiments, a tertiary amide has the following formula: 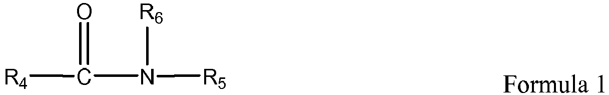 wherein
R₄ is a saturated fatty group having 11 - 60 carbon atoms
R₅ and R₆ are independently lower alkyl, aryl, aryl lower alkyl or saturated fatty group of 11 - 29 carbon atoms or R₇, where R₇ is :

   R₁-Ar-O-R₂-O-R₃
R₁ is an alkyl group containing 1 - 15 carbon atoms;
R₂ and R₃ are independently lower alkyl groups containing 1 - 6 carbon atoms and Ar is an aryl group.

The term "lower alkyl", when used alone or in combination, means an alkyl group containing 1-6 carbon atoms. The lower alkyl group may be branched or straight chained. Preferred lower alkyl contains 1-4 carbon atoms and more preferably 1 or 2 carbon atoms. Examples of lower alkyl include methyl, ethyl, propyl, isopropyl, butyl, t-butyl, isobutyl, sec-butyl, pentyl and hexyl.

As used herein, the term "aryl", when used alone or in combination, is an aromatic group comprised solely of carbon ring atoms. The aryl group may be monocyclic, bicyclic or tricyclic. If more than 1 ring is present, the rings are fused; thus the aryl group also includes polynuclear aromatics, i.e., bicyclic and tricyclic fused aromatic rings. The aryl group contains 4n+2 ring carbon atoms, wherein n is 1-4. The aryl group contains 6, 10, 14 or 18 ring carbon atoms and up to a total of 25 carbon atoms. It is preferred that n is 1-3. In an embodiment, aryl groups are phenyl, naphthalene, including alpha and beta-naphthalene, anthracene, phenantluvne, and the like. In another embodiment the aryl group is naphthalene or phenyl, and in still another embodiment, the aryl group is phenyl.

The aryl group may be unsubstituted or substituted with one or more electron donating groups or electron withdrawing groups. Terms "electron withdrawing groups" and "electron donating groups" refer to the ability of a substitutent to withdraw or donate electrons relative to that of hydrogen if the hydrogen atom occupied the same position in the molecule. These terms are well understood by one skilled in the art and are discussed in Advanced Organic Chemistry, by J. March, 4th Ed. John Wiley and Sons, New York, N.Y. pp. 16-18 (1992). Examples of electron withdrawing groups include halo, especially fluoro, bromo, chloro, iodo, and the like; nitro; carboxy; formyl; lower alkanoyl; carboxyamido; triloweralkylamino; aryl; trifluoromethyl; aryl lower alkanoyl; lower carbalkoxy; and the like. Examples of electron donating groups include such groups as hydroxy; lower alkoxy, including methoxy, ethoxy, and the like; lower alkyl; amino; lower alkylamino; dilowerlakylamino; aryloxy (such as phenoxy); mercapto; mercapto lower alkyl; lower alkylthio; and the like. One skilled in the art will appreciate that the aforesaid substituents may have electron donating properties under one set of circumstances and electron withdrawing properties under different chemical conditions or circumstances; these are also contemplated to be within the scope of these terms. Moreover, the present invention contemplates any combination of substituents selected from the above-identified terms.

In an embodiment, the aryl group is unsubstituted or substituted by lower alkyl groups.

The term "aryl lower alkyl group" refers to a lower alkyl group as defined herein bridging an aryl group, as defined herein, to the main chain. Examples include benzyl, phenethyl, phenpropyl, phenisopropyl, phenbutyl, diphenyl methyl, 1,1-diphenylethyl, 1, 2- diphenylethyl, and the like.

The fatty acid or fatty alcohol, as used herein, is a saturated aliphatic which may be straight or branched chain. It is preferred that the fatty acid contains 12-30 carbon atoms and more preferably 16-22 carbon atoms and most preferably 16-20 carbon atoms. Examples include lauric acid, myristic acid, palmitic acid, stearic acid, behenic acid, arachidic acid, and the like.

As used herein, the term "fatty group", when used alone or in combination is a fatty acid group without the terminal carboxy moiety on the omega carbon of the chain.

In other words, it is R₈ wherein 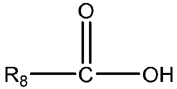 is the corresponding fatty acid. The fatty group (R₈) contains 11-29 carbon atoms and more preferably contains an odd number of carbon atoms. In an embodiment, it contains 15-21 carbon atoms. All of the carbon-carbon bonds in R8 are saturated. It may be straight chained or branched.

In an embodiment, the tertiary amides used have the formula 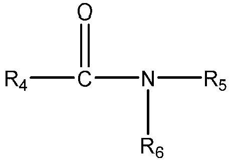 or pharmaceutically acceptable salts thereof.
R₄ is a saturated fatty group containing 11-60 carbon atoms. R₅ and R₆ are independently aryl, aryl lower alkyl, a saturated fatty group containing 11-29 carbon atoms, or R₇ where R₇ is

R₁-Ar-O-R₂-O-R₃

R₂ and R₃ are lower alkyl groups containing 1-6 carbon atoms, R₁ is a lower alkyl group and Ar is aryl.

These tertiary amides and hydrates are described in U.S. Patent Publication No. 2004/0122105. However, it is to be understood that the tertiary amide or hydrate cannot have any groups thereon that can react with free iodine, such as carbon-carbon double bonds unless they are part of an aromatic ring, as described herein.

The R₄ in an embodiment is an aliphatic containing 15-46 carbon atoms. The R₄ group is completely saturated.

In an embodiment R₂ and R₃ contains 1-3 carbon atoms and more preferably 1 or 2 carbon atoms. It is also preferred that R₂ and R₃ are the same. It is even more preferred that R₂ and R₃ are the same and contain 1 or 2 carbon atoms and most especially 2 carbon atoms. In an embodiment aryl is phenyl.

In an embodiment R₇ is 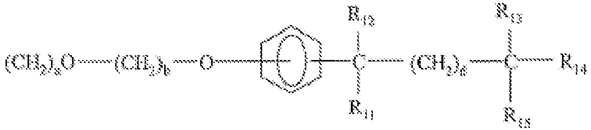 wherein R₁₁, R₁₂, R₁₃, R₁₄, R₁₅ are independently lower alkyl and a, b, d are independently 1-5.

In an embodiment, R₁₁ and R₁₂ are the same and R₁₃, R₁₄ and R₁₅ are the same. In another embodiment, R₁₂, R₁₁R₁₃, R₁₄ and R₁₅ are the same.

In another embodiment, a, b and d are independently 1-3. In another embodiment, a and b are the same. In a still another embodiment a is 2, b is 3 and d is 1.

In another embodiment R₇ is

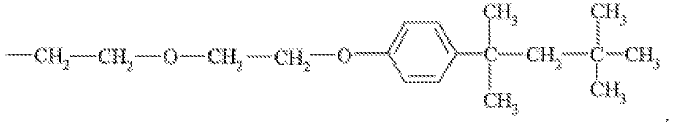

R₁ is, in an embodiment, an alkyl group containing 1-10 carbon atoms and in another embodiment containing 1-6 carbon atoms. It may be straight chained or branched.

In still another embodiment, R₅ and R₆ are both independently saturated fatty groups or one of R₅ and R₆ is an aryl or aryl lower alkyl and the other is R₇ or one of R₅ and R₆ is a saturated fatty group and the other is R₇, or one of R₅ and R₆ is a saturated fatty group and the other is an aryl or aryl lower alkyl.

In an embodiment, the tertiary amide is benzethonium stearamide or benzalkonium stearamide, i.e., compounds having the formula respectively, 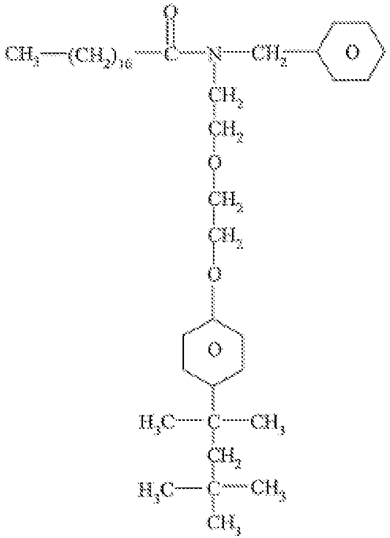 or 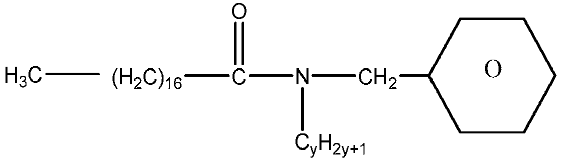 where y is 8, 10, 12, 14, 16 or 18.

In an embodiment y is 12 or 14.

In another embodiment the second component is a mixture of one or more tertiary amides or one or more tertiary amide hydrates or one or more salts of either or a combination thereof.

The second component, the tertiary amide or salt thereof or the tertiary amide hydrate or salts thereof are present in an amount effective to form a film over the area of the skin to be treated, as described below. In an embodiment, the second component is present in an amount ranging from about 0.1% to about 2% by weight of the pharmaceutical composition and in another embodiment, from about 0.5% to about 1.5% by weight of the pharmaceutical composition.

### Low Iodine Reactive Compounds

In some embodiments, provided compositions include a low iodine reactive compound, specifically a low iodine reactive emollient. As describe above, low iodine reactive compounds have an iodine number lower than about 80 (e.g. less than about 70, less than about 60, less than about 50, less than about 40, less than about 30, less than about 25, less than about 20, less than about 15, less than about 10, less than about 5, less than about 2, about 0). As an emollient these compounds have the effect of slowing water evaporation from the skin, thus softening or soothing the skin. Traditional emollients include common fats and oils, vegetable oils, mineral oils, petrolatum, lanolin, dimethicone, synthetic oils and lubricants, but many of these are reactive with respect to iodine, thus not necessarily suitable as a sole emollient for the compositions described herein.

Exemplary low iodine reactive compounds, specifically emollients, include, but are not limited to petrolatum, mineral oil, mineral wax, fully hydrogenated vegetable oils, lard, tallow, jojoba oil, hydrogenated jojoba esters, babassu oil, coconut oil, cocoa butter, macadamia oil, olive oil, peanut oil, nabar seed oil, and combinations and derivatives thereof. Further low iodine reactive compounds include but are not limited to hydrogentated polybutenes, isododecane and related hydrocarbons, certain silicones, C8 - C10 trigycerides, glyceryl tribehenate, hydrogenated castor oil, palm oil, some esters made with saturated fats and combinations thereof.

Jojoba oil, in particular, has a very low iodine number as well as a number of feature that lend itself to use in topical compositions. Jojoba oil is a mixture of wax esters (about 36 - 46 carbons in length. Typically jojoba oil contains about 66 to about 76 weight percent eisconsenoic acid; about 14 weight percent to about 20 weight percent docosenoic acid and about 10 weight percent to about 13 weight percent oleic acid. Derivatives of jojoba oil (e.g. hydrogenated jojoba esters) can also be used as an emollient. Other derivatives of jojoba oil include jojoba esters, isopropyl jojobate, jojoba alcohol, hydrolyzed jojoba esters, jojoba wax PEG-120 esters, jojoba oil PEG-150 esters, jojoba wax PEG-80 esters and jojoba glaze.

### Additional Components

In some embodiments, provided compositions include one or more additional components. For examples, in some embodiments, provided compositions contain one ore more surfactant(s). The composition can contain one surfactant or a combination of two or more surfactants. The surfactant can be an anionic surfactant, a cationic surfactant, an amphoteric surfactant or a non-ionic surfactant or a combination thereof of two or more surfactants in the same class or in different classes.

Examples of anionic surfactants include fatty acid soaps (e.g., sodium laurate, TEA stearate, sodium palmitate and the like); higher alkyl sulfates (e.g., sodium lauryl sulfate, potassium lauryl sulfate and the like); alkyl ether sulfates (e.g., triethanolamine POE-lauryl sulfate, sodium POE lauryl sulfate and the like); N-acylsarcosinic acids(e.g., sodium lauroylsarcosinate and the like); higher fatty acid amide sulfates (e.g., sodium N-myristoyl-N-methyltaurate, sodium cocoyl methyltaurate, sodium lauroyl methyltaurate and the like); phosphates (sodium POE oleyl ether phosphate, POE-stearyl ether phosphoric acid and the like); sulfosuccinates (e.g., sodium di-2-ethylhexylsulfosuccinate, sodium monolauroyl monoethanolamide polyoxyethylenesulfosuccinate, sodium lauryl polypropyleneglycol sulfosuccinate and the like); alkylbenzenesulfonates (e.g., sodium linear dodecylbenzenesulfonate, triethanolamine linear dodecylbenzenesulfonate, linear dodecylbenzenesulfonic acid and the like); higher fatty acid ester sulfates (e.g., sodium hydrogenated cocoglyceride sulfate and the like); N-acylglutamates (e.g., monosodium N-lauroylglutamate, disodium N-stearoylglutamate, monosodium N-myristoyl-L-glutamate and the like); sulfated oils (e.g., turkey red oil and the like); POE alkyl ether carboxylic acids; POE alkyl allyl ether carboxylates; alpha-olefinsulfonates; higher fatty acid ester sulfonates; secondary alcohol sulfates; higher fatty acid alkylolamide sulfate; sodium lauroyl monoethanolamide succinate; ditriethanolamine N-palmitoylaspartate; sodium caseinate and the like.

Examples of cationic surfactants include alkyl trimethyl ammonium salts (e.g., stearyl trimethyl ammonium chloride, lauryl trimethyl ammonium chloride and the like); alkyl pyridinium salts (e.g., cetylpyridinium chloride and the like); distearyl dimethyl ammonium chloride ; poly(N,N-dimethyl-3,5-methylenepiperidinium) chloride; alkyl quaternary ammonium salts; alkyl dimethyl benzyl ammonium salts; alkylisoquinolinium salts; dialkylmorphonium salts; POE alkylamines; alkylamine salts; polyamine fatty acid derivatives; amyl alcohol fatty acid derivatives; benzalkonium chloride; benzethonium chloride and the like.

Examples of amphoteric surfactants include imidazoline series amphoteric surfactants (e.g., 2-undecyl-N,N,N-(hydroxyethylcarboxymethyl)-2-imidazoline sodium, 2- cocoyl-2-imidazolinium hydroxide-1-carboxyethyloxy disodium salt and the like); betaine series surfactants (e.g., 2-heptadecyl-N-carboxymethyl-N-hydroxyethylimidazolini-um betaine, lauryldimethylaminoacetic acid betaine, alkylbetaine, amidebetaine, sulfobetaine and the like) and the like.

Examples of lipophilic nonionic surfactants include sorbitan fatty acid esters (e.g., sorbitan monooleate, sorbitan monoisostearate, sorbitan monolaurate, sorbitan monopalmitate, sorbitan monostearate, penta-2-ethylhexylic acid diglycerol sorbitan, tetra-2-ethylhexylic acid diglycerol sorbitan and the like); glycerin polyglycerin fatty acids (e.g., mono-cottonseed oil fatty acid glycerin, monostearic acid glycerin, monostearic acid glycerin malic acid and the like); propylene glycol fatty acid esters (e.g., monostearic acid propylene glycol and the like); hydrogenated castor oil derivatives; glycerin alkyl ethers and the like.

Examples of hydrophilic nonionic surfactants include POB-sorbitan fatty acid esters (e.g., POE-sorbitan monostearate, POE-sorbitan monooleate, POB-sorbitan tetraoleate and the like); POE-sorbit fatty acid esters (e.g., POE-sorbitol monolaurate, POE-sorbitol monostearate and the like); POE-glycerin fatty acid esters (e.g., POE-glyceryl monostearate, POE-glyceryl monoisostearate, and POE-glyceryl triisostearate); POE-fatty acid esters (e.g., POE-distearate, ethylene glycol distearate and the like); POE-alkyl ethers(e.g., POE-lauryl ether, POE-stearyl ether, POE-behenyl ether, POE-2-octyldodecyl ether, POE-cholestanol ether and the like); Pluronic type surfactants (e.g., Pluronic and the like); POE.POP-alkyl ethers (e.g., POE.POP-cetyl ether, POE.POP-2-decyltetradecyl ether, POE.POP-monobutyl ether, POE.POP-hydrogenated lanolin, POE.POP-glyceryl ether and the like); fused tetraPOE.tetraPOP-ethylenediamines (e.g., Tetronic and the like); alkanolamides (e.g., coconut oil fatty acid diethanolamide, lauric acid monoethanolamide, fatty acid isopropanolamide and the like); POE-propylene glycol fatty acid esters; POE-alkylamines; POE-fatty acid amides; sucrose fatty acid esters; alkylethoxydimethylamine oxides;trioleylphosphoric acid and the like. POE, hereinabove, is polyoxyethylene.

In an embodiment, the composition contains non-ionic surfactants and quaternary cationic surfactants. Examples include Triton-X-100, Nonoxynol NP-9, Arosurf TA-100 or a combination of two or all three, and the like.

The composition may contain 1 surfactant or a combination of surfactants. The surfactant(s) are present in an emulsifying effective amount. In an embodiment, the surfactants to present in total in an amount ranging from about 2% to about 10% by weight and in another embodiment, from about 5% to about 8% by weight of the pharmaceutical composition and most preferably from 6.5% to 7.5%,

The pharmaceutical composition is prepared by incorporating the aforementioned ingredients into a lulown base for a topical composition for skin. The pharmaceutical composition is mixed with the ingredients normally used in a cosmetic or medical topical composition, according to the conventional methods. Examples of ingredients normally found include powders, lipid fat or oil, solid fats or oil, waxes, hydrocarbons, higher fatty acids, higher alcohols, esters, additional surfactants, water-soluble polymers, thickeners, film forming agents, ultraviolet absorbing agents, metal ion sequestering agents, lower alcohols, multivalent alcohols, pH adjusting agents, water, skin softeners, skin humectants and the like.

The composition may additionally contain a skin softener. Examples include allantoin, and comfrey extract (See U.S. Patent. No. 6,583,184) and water lily extracts (See U.S. Patent No. 6,878,378) and the like. If present, it is present in skin softening effective amounts. In an embodiment, it is present in an amount ranging from about 0.5 % to about 10% by weight of the pharmaceutical composition and in another embodiment, it is present in about 1% to about 3% by weight of the composition.

Examples of powders include inorganic powders, e.g., talc, kaolin, mica, sericite, muscovite, phlogopite, synthetic mica, red mica, biotite, vermiculite, magnesium carbonate, calcium carbonate, aluminum s licate, barium silicate, calcium silicate, magnesium silicate, strontium silicate, tungsten acid metal salt, magnesium, silica, zeolite, barium sulfate, calcinated calcium sulfate (calcined gypsum), calcium phosphate, fluorine apatite, hydroxyapatite, ceramic powder, metal soap (e.g., zinc myristate, calcium palmitate, aluminium stearate), boron nitride and the like); organic powders, e.g., polyamideresin powder (nylon powder), polyethylene powder, polystyrene powder, polyethylene tetrafluoride powder and the like, inorganic white pigments (e.g., titanium dioxide, zinc oxide and the like); inorganic red series pigments (e.g., iron oxide (red iron oxide), iron titanate and the like); inorganic brown series pigments (e.g., gamma-iron oxide and the like); inorganic yellow series pigments (e.g., yellow iron oxide, bess and the like); inorganic black series pigments (e.g., black iron oxide, lower titanium oxide and the like); inorganic purple series pigments (e.g., mangoviolet, cobaltviolet and the like); inorganic green series pigments (e.g., chromium oxide, chromium hydroxide, cobalt titanate and the like); inorganic blue series pigments (e.g., ultramarine, Prussian blue and the like); pearl pigments (e.g., titanium oxide-coated mica, titanium oxide-coated bismuth oxychloride, titanium oxide-coated talc, colored titanium oxide-coated mica, bismuth oxychloride, fish scale flake and the like); metal powder pigments (e.g., aluminum powder, copper powder) and the like.

Examples of waxes include beeswax, candelilla wax, cotton wax, carnauba wax, bayberry wax, insect wax, spermaceti, montan wax, bran wax, kapok wax, lanolin acetate, liquid lanolin, sugarcane wax, lanolin fatty acid isopropyl, hexyl laurate, jojoba wax, shellac wax, and the like. If a wax is present, in an embodiment, it is present in amounts ranging from about .5 % to about 2% by weight of the pharmaceutical composition, and in another embodiment, from about 1 to about 1.5% by weight of the pharmaceutical composition.

Examples of the hydrocarbon oil include liquid paraffin, ozokerite, squalane, pristane, and paraffin, ceresin, squalene, vaseline, microcrystalline wax and the like.

Examples of the higher fatty acid include lauric acid, myristic acid, palmitic acid, stearic acid, behenic acid, acid, tallic acid, isostearic acid, and the like.

Examples of the higher alcohols include straight alcohols (e.g., lauryl alcohol, cetyl alcohol, stearyl alcohol, myristyl alcohol, cetostearyl alcohol and the like); branched alcohols (e.g., monostearylglycerin ether (batyl alcohol), 2-decyltetradecinol, hexyldodecanol, isostearyl alcohol, octyldodecanol and the like) and the like.

Examples of the synthetic ester oil include isopropyl myristate, cetyl octanoate, octyldodecyl myristate, isopropyl palmitate, butyl stearate, hexyl laurate, myristyl myristate, hexyldecyl dimethyloctanoate, cetyl lactate, myristyl lactate, isocetyl stearate, isocetyl isostearate, di-2-ethylhexanoic acid ethylene glycol, dipentaerythritol fatty acid ester, monoisostearic acid N-alkyl glycol, dicapric acid neopentyl glycol, diisostearyl malate, di-2-heptylundecanoic acid glycerin, tri-2-ethylhexanoic acid trimethylolpropane, triisostearic acid trimethylolpropane, tetra-2-ethylhexanoic acid pentaerythritol, tri-2-ethylhexanoic acid glycerin, trioctanoic acid glycerin, triisopalmitic acid glycerin, triisostearic acid trimethylolpropane, cetyl 2-ethylhexanoate, 2-ethylhexyl palmitate, trimyristic acid glycerin, tri-2-heptylundecanoic acid glyceride, acetoglyceride, 2-heptylundecyl palmitate, diisobutyl adipate, N-lauroyl-L-glutamic hhacid-2-octyldodecyl ester, di-2-heptylundecyl adipate, ethyl laurate, 2-hexyldecyl myristate, 2-hexyldecyl palmitate, 2-hexyldecyl adipate, diisopropyl sebacate, 2-ethylhexyl succinate, triethyl citrate and the like.

Examples of skin humecants are aloe extract, urea, 1,3-butyleneglycol, glycerin, propylene glycol, sodium pyrrolidone carboxylate, and the like.

Examples of thickeners include gum Arabic, carrageenan, karaka gum, tragacanth gum, carob gum, quinceseed (Cydonia oblonga), caseine, dextrin, gelatin, sodium pectinate, sodium alginate, methylcellulose, ethylcellulose, CMC, hydroxyethylcellulose, hydroxypropylcellulose, PVA, PVM, PVP, sodiuin polyacrylate, carboxyvinyl polymer, amigel, locust bean gum, guar gum,tamarind gum, dialkyldimethylammonium sulfate cellulose, xanthan gum, aluminium magnesium silicate, bentonite, hectorite, AlMg silicate (Veegum), laponite amigel, anhydrous silicic acid and the like. If present, it is present in gelling effective amounts. In an embodiment, it is present, it is present from about 0.25% by weight to about 1.25 % by weight.

Examples of ultraviolet absorbing agents include benzoic acid series ultraviolet absorbing agents (e.g., paraaminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester and the like); anthran lie acid series ultraviolet absorbing agents (e.g., homomenthyl-N-acetyl anth^{•}anilate and the like); salicylic acid series ultraviolet absorbing agents (e.g., amyl salicylate, menthyl salicylate, homomenthyl salicylate, octyl salicylate, phenyl salicylate, benzyl salicylate, p-isopropanol phenyl salicylate and the like); sinnamic acid series ultraviolet absorbing agents (e.g., octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-pmethoxy cinnamate, octyl-p-methoxy cinnamate (2-ethylhexyl-p-methoxy cinnamate), 2- ethoxyethyl-p-methoxy cinnamate, cyclohexyl-p-methoxy cinnamate, ethyl-alpha-cyanobeta-phenyl cinnamate, 2-ethylhexyl-alpha-cyano-beta-phenyl cinnamate, glyceryl mono-2-ethylhexanoyl-diparamethoxy cinnamate and the like); benzophenone series ultraviolet absorbing agents (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid salt, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone and the like); 3-(4'-methylbenzylidene)-d,l -camphor, 3-benzylidene-d,l-camphor; 2-pheny1-5- methylbenzoxazole; 2,2'-hydroxy-5-methylphenylbenzotriazole; 242'-hydroxy-5¹-toctylphenyl)benzotriazole; 2-(2'-hydroxy-5'-methylphenylbenzotriazole; dibenzaladine; dianisoylmethane; 4-methoxy-4'-t-butyldibenzoylmethane; 5-(3,3-dimethyl-2-norbomylidene)-3-pentane-2-one and the like.

Examples of sequestering agents include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium methaphosphate, gluconic acid, phosphoric acid, citric acid, ascorbic acid, succinic acid, edetic acid, trisodium ethylenediaminehydroxyethyltriacetate, EDTA, or salt thereof, and the like. If present, it is present in less than about 1% by weight of the pharmaceutical composition.

Examples of lower alcohol include ethanol, propanol, isopropanol, isobutyl alcohol, t-butyl alcohol and the like.

Examples of polyols include diols (e.g., ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol and the like); triols (e.g., glycerin trimethylolpropane and the like); tetraols (e.g., pentaerythritol such as 1,2,6-hexanetriol and the like); pentaols (e.g., xylitol and the like); hexaols (sorbitol, mannitol and the like); polyol polymers (e.g., diethylene glycol, dipropylene glycol, triethylene glycol, polypropylene glycol, tetraethylene glycol, diglycerin, polyethylene glycol, triglycerin, tetraglycerin, polyglycerin and the like); diol alkyl ethers (e.g., ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether and the like); diol alkyl ethers (e.g., diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether and the like); diol ether ester (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate and the like); glycerin monoalkyl ether (e.g., chimyl alcohol, selachyl alcohol, batyl alcohol and the like); glyceride; tetrahydrofurfiiryl alcohol; POE-tetrahydrofurfuryl alcohol; POP-butyl ether; POP.POE-butyl ether; tripolyoxypropylene glycerin ether; POP-glycerin ether; POP-glycerin ether phosphoric acid; POP.POE-pentaerythritol ether, polyglycerin and the like.

Another optional ingredient is a skin protectant. A skin protectant agent is an agent which protects injured or exposed skin or mucous membrane surfaces from harmful or annoying stimuli and includes, but is not limited to, allantoin, calamine, cocoa butter, dimethicone, glycerin, kaolin, petrolatum, shark liver oil, silicates, silicas, protectant clays, zinc acetate, zinc carbonate, and zinc oxide. Skin protectant agents are well known in the art. One of ordinary skill in the art would understand, appreciate and recognize agents that are considered to be skin protectant agents. An extensive list of skin protectant agents can be found in the International Cosmetic Ingredient Dictionary and Handbook published by the Cosmetic, Toiletry, and Fragrance Association, Inc., under the listing "Skin Protectants." If present, it is present in skin protectant amounts. In an embodiment, it is present in amounts ranging from about 0.1 to about 1% by weight, and in another embodiment, from about 0.4 to about 0.7 weight percent of the pharmaceutical composition.

The present pharmaceutical composition may optionally contain preservatives typically used in pharmaceutical and cosmetic compositions. Examples include methyl paraben, butyl paraben, ethyl paraben, sorbic acid, sodium benzoate, and the like. The pharmaceutical composition may additionally contain other microbicides. Examples include lauricidin and Merguard 1200. Additional preservatives or microbicides, if present, range from about 1% to about 9 % by weight, and more preferably from about 4 % to about 7 % by weight of the pharmaceutical composition.

The pharmaceutical composition has a pH less than 3.5. In an embodiment, the pH ranges from about 2.0 to about 3.5. In another embodiment, the pH range from about 3.0 to about 3.3.

The pH of the composition does not require pH adjusting agents, however, buffers can be utilized to maintain the pH in the ranges described hereinabove.

The specific gravity of the composition is greater than about 0.9 and less than about 1.1. In an embodiment, it is greater than about 0.95 and in another embodiment, it ranges from about 0.95 to about 1.05.

The present pharmaceutical composition contains water. The amount of water present varies, but typically it contains at least about 60% by weight water. In an embodiment, it contains about 50 to about 70% water and in another embodiment, from about 57 to about 62% water.

In an embodiment, the pharmaceutical composition is comprised of a povidoneiodine complex having from about 8% to about 12% available iodine by weight in an amount ranging from about 1% to about 15% of the pharmaceutical composition. It contains one or more non-ionic surfactants and at least one quaternary ammonium surfactant in emulsifying effective amounts. It also contains coconut oil in the amount described herein.

In another embodiment, the composition contains povidone-iodine in microbicidal effective amounts; tertiary amide in film forming effective amounts; at least one quaternary and at least one non-ionic surfactant in emulsifying effective amounts; low iodine number emollients in the amount described hereinabove, and optionally beeswax in film forming effective amounts; and optionally at least one of the following: a metal chelating agent in a chelating effective amount, e.g., a skin humectant in an humectant effective amount; a skin protectant in a skin protectant effective amount; an organic base in acid neutralizing effective amounts; a nonionic detergent in effective amounts; a thickener in gelling effective amounts; and/or additional microcides (such as preservatives). In another embodiment, it contains all of the aforementioned optional components in addition to the povidone iodine, surfactants and coconut oil.

### Forms & Use

Suitable dosage forms for the described compositions includes, but are not limited to creams, ointments, lotions, gels, powders, sprays, aerosols, foams, pastes, putties, liquids, very thin emulsions (i.e., milks) or any one of a variety of topical forms for used in topical administration.

Ointments, as known in the field, are semisolid preparations that are typically based on petrolatum or other petroleum derivatives. The specific ointment foundation to be used is one that will provide for optimum delivery of povidone-iodine and also other desired characteristics (e.g., emolliency without reacting with iodine). In general, ointment foundations may be grouped into four classes, oleaginous, emulsifiable, emulsion and water-soluble. Oleaginous ointment foundation, for example, includes vegetable oils, fats obtained from animals, and semisolid hydrocarbons obtained from petroleum. Emulsifiable ointment foundations also known as absorbent ointment foundations contain little or no water and include, e.g., hydroxystearin sulfate, anhydrous lanolin and hydrophilic petrolatum. Emulsion ointment foundations are either water-in-oil (W/O) emulsions or oil-in-water (O/W) emulsions and include, for example, ethyl alcohol, glyceryl monostearate, lanolin and stearic acid.

Creams, also known in the art, are viscous liquids or semisolid emulsions, either oil-in-water or water-in-oil. Cream foundations are water-washable and typically contain an oil phase, an emulsifier and an aqueous. The oil phase, also called, the "internal" phase, is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation is generally a nonionic, anionic cationic or amphoteric surfactant.

Gels, as used herein, are semisolid, suspension-type systems. Single-phase gels contain organic macromolecules distributed substantially uniformly throughout the carrier liquid, which is typically aqueous, but also contains an alcohol and, optionally, an oil. The gelling agents may be crosslinked acrylic acid polymers such as the "carbomer" family of polymers, e.g., carboxypolyalkylenes that may be obtained commercially (e.g. Carbopol™). Also hydrophilic polymers such as polyethylene oxides, polyoxyethylene-polyoxypropylene copolymers and polyvinylalcohol; cellulosic polymers such as hydroxypropyl cellulose, hydroxyethyl cellulose, hydroxypropyl methylcellulose, hydroxypropyl methylcellulose phthalate, and methyl cellulose; gums such as tragacanth and xanthan gum; sodium alginate; and gelatin and amigel are preferred gels. In order to prepare a uniform gel, dispersing agents such as alcohol or glycerin can be added, or the gelling agent can be dispersed by trituration, mechanical mixing or stirring, or combinations thereof.

Lotions are preparations to be applied to the skin surface without friction, and are typically liquid or semi-liquid preparations in which solid particles, including the active agent are present in a water or alcohol base. Lotions are usually suspensions of solids that comprise a liquid oily emulsion of the oil-in-water type. Lotions can be used to treat large body areas, because of the ease of applying a more fluid composition. It is generally necessary that the insoluble matter in a lotion be finely divided. Lotions will typically contain suspending agent to produce better dispersions as well as compounds useful for localizing and holding the active agent in contact with the skin, e.g., methylcellulose, sodium carboxymethylcellulose or the like.

Pastes are semisolid forms in which the active (e.g., povidone-iodine) is suspended in a suitable foundation. Depending on the nature of the foundation, pastes are divided between fatty pastes or those made from single-phase, aqueous gels. The foundation in a fatty paste is generally petrolatum or hydrophilic petrolatum or the like. The pastes made from single-phase aqueous gels generally incorporate carboxymethylcellulose or the like as the foundation.

The delivery system for the dosage form may use either a pump or propellants, such as hydrocarbons, hydrofluorocarbons, nitrogen, nitrous oxide, carbon dioxide or ethers, for example dimethyl ether to dispense the composition. In some embodiments, the gas is oxygen free. In some embodiments, the delivery system is in a single phase system as this allows less complicated manufacture.

Various additional delivery systems can be utilized, depending on the end use. An embodiment for hand sanitizing is a bag-in-box of wall mounted dispenser. Pump containers and tubes may also be utilized. For wound antiseptic use, an aerosolized can or a bag-on-valve (BOV) can may be used. This permits antiseptic to be pushed into the wound. In another embodiment for hand delivery is a unit dose that allows completed coverage economically.

As described above, in a further aspect the present invention describes methods of use of the above described compositions, also described in more detail below. The method of use removes microbes (e.g., bacteria) from the skin of a subject (or the surface of an object). To do so, an antiseptically effective amount of the described composition is applied to an area of the skin, thoroughly rubbed into and on the skin to substantially cover the area of the skin to be disinfected.

In application, an aliquot comprising antiseptically effective amount of the composition is applied to the area of skin to cover the area which is to be cleansed from microbes. In an embodiment, at least about 0.01 g per square inch of the composition is placed on to the area of the skin (e.g., the hands). In some embodiments, 0.01 g to about 0.03 g per square inch of the composition is applied to the area of the skin to be cleansed of microbes. In some embodiments, 0.1 grams to 1 gram per square inch is applied. Excess amounts of the composition can be used, and excess amounts can be washed off or wiped off, from the skin. The composition is spread so that it completely covers the area to be cleansed, e.g., spread over all surface of the hands or feet. The composition is rubbed into and on the skin until dry. The composition is allowed to remain on the skin for a sufficient time to kill the microbes thereon. In some embodiments, the composition remains on the skin for at least about 30 seconds, for at least about 120 seconds, for at least about 1 minute, for at least about 2 minutes, for at least about 5 minutes, and any ranges there between. Then the composition may be rinsed with water. The method can be repeated as necessary or at regular intervals (e.g., every 1, 2, 3, 4, or 6 hours.). The compositions can also be used for protracted periods of time without having to be removed, such as during long surgeries, under gloves or surgical draping, or for first-responder field use with or without gloves.

Without being bound by any theory, it is believed that short chain fatty acids in the composition are absorbed by the skin, through the film formed by the tertiary amide. The detergents in the composition, freed from the fats, then interact with dirt and microbes on the skin, and long chain fats that are not absorbed by the skin form an occlusive layer over the skin. The occlusive layer helps the skin retain its natural moisture

Unreacted cationic surfactants emulsify waxes in the composition, long chain fats and povidone to form a positively-charged occlusive layer. This layer is last-to-dry and is electrostatically repelled by the acid hydrate film. Between these two films is a layer made of preservatives and nonionic detergents. After drying, excess preservative and detergent is rinsed away, assuring a persistent, reproducible layer captured between the proximal acid hydrate film and the distal cationic waxy film. The addition of the long chain fats from the emollient provide the distal film with a cosmetically elegant hand feel after rinsing and drying. The multiple layers make the compositions antimicrobial activity persistent for up to about 6 hours.

### Examples

### Example 1

An aqueous phase was first prepared by mixing the following components in the weight proportions listed. The aqueous phase was heated to 70 °C and mixed.

| Name of Ingredient | Weight Percentage (of overall composition) |
|---|---|
| Distilled Water, USP | 58.35 |
| Stearic Acid, NF | 3.50 |
| Benzalkonium Chloride | 0.10 |

Next the second phase was added, after which mixing and a temperature of 70 °C were maintained for 30 minutes.

| Name of Ingredient | Weight Percentage (of overall composition) |
|---|---|
| Cetyl Alcohol | 3.00 |

Next, the third phase was added. Each ingredient of the third phase, listed in the table below, was added in sequence while mixing and a temperature of 70 °C were maintained in the mixing vessel. The mixture was mixed for 10 minutes before the next addition commenced.

| Name of Ingredient | Weight Percentage (of overall composition) |
|---|---|
| Triton X-100 | 3.00 |
| Nonoxynol NP9 | 2.55 |
| Merquat 550 | 1.40 |
| Trolamine NF | 0.75 |
| Varisoft TA-100 | 1.70 |
| PVP-USP/NF | 0.50 |
| EDTA | 0.20 |
| Ajidew N100 | 0.10 |

Next, the fourth phase was added. Each ingredient in the table below was added in sequence while mixing an a temperature of 70 °C were maintained. The mixture was mixed for 10 minutes.

| Name of Ingredient | Weight Percentage (of overall composition) |
|---|---|
| Lauricidin | 4.50 |
| Floraester | 1.20 |

After 10 minutes of mixing, the second part of the fourth phase was added. Each of the ingredients in the table below were added in sequence. After the last ingredient was dissolved, heat was removed from the mixture.

| Name of Ingredient | Weight Percentage (of overall composition) |
|---|---|
| Jeechem GC | 1.50 |
| Arlacel 165 | 2.75 |

The fifth phase is then added. Each of the ingredients in the table below are added in sequence:

| Name of Ingredient | Weight Percentage (of overall composition) |
|---|---|
| Allantoin | 0.40 |
| Butylene Glycol | 4.50 |

The mixture is allowed to 58 °C after which the sixth phase is added. The sixth phase comprises the ingredients in the following table:

| Name of Ingredient | Weight Percentage (of overall composition) |
|---|---|
| Amigel | 0.50 |

After the sixth phase is mixed thoroughly into the composition, the mixture continues to cool. Once the mixture cools to 50 °C, the seventh phase is added. The seventh phase comprises the ingredients in the following table:

| Name of Ingredient | Weight Percentage (of overall composition) |
|---|---|
| PVP-I | 7.50 |

Once the ingredients of the seventh phase are dissolved, the eighth phase is added, the eighth phase comprising the following ingredients:

| Name of Ingredient | Weight Percentage (of overall composition) |
|---|---|
| Sepigel | 2.00 |

### Example 2

An aqueous phase was first prepared by mixing the following components in the weight proportions listed. The aqueous phase was heated to 70 °C and mixed.

| Name of Ingredient | Weight Percentage (of overall composition) |
|---|---|
| Distilled Water, USP | 61.30 |
| Stearic Acid, NF | 3.00 |
| Benzalkonium Chloride | 0.10 |

Next the second phase was added, after which mixing and a temperature of 70 °C were maintained for 30 minutes.

| Name of Ingredient | Weight Percentage (of overall composition) |
|---|---|
| Cetyl Alcohol | 2.50 |

Next, the third phase was added. Each ingredient of the third phase, listed in the table below, was added in sequence while mixing and a temperature of 70 °C were maintained in the mixing vessel. The mixture was mixed for 10 minutes before the next addition commenced.

| Name of Ingredient | Weight Percentage (of overall composition) |
|---|---|
| Triton X-100 | 3.00 |
| Nonoxynol NP9 | 3.00 |
| Merquat 550 | 1.40 |
| Trolamine NF | 0.70 |
| Varisoft TA-100 | 1.70 |
| PVP-USP/NF | 0.30 |
| EDTA | 0.20 |
| Ajidew N100 | 0.10 |

Next, the fourth phase was added. Each ingredient in the table below was added in sequence while mixing an a temperature of 70 °C were maintained. The mixture was mixed for 10 minutes.

| Name of Ingredient | Weight Percentage (of overall composition) |
|---|---|
| Lauricidin | 4.00 |
| Floraester | 1.50 |

After 10 minutes of mixing, the second part of the fourth phase was added. Each of the ingredients in the table below were added in sequence. After the last ingredient was dissolved, heat was removed from the mixture.

| Name of Ingredient | Weight Percentage (of overall composition) |
|---|---|
| Jeechem GC | 1.50 |
| Arlacel 165 | 2.30 |

The fifth phase is then added. Each of the ingredients in the table below are added in sequence:

| Name of Ingredient | Weight Percentage (of overall composition) |
|---|---|
| Allantoin | 0.40 |
| Butylene Glycol | 3.00 |

The mixture is allowed to 58 °C after which the sixth phase is added. The sixth phase comprises the ingredients in the following table:

| Name of Ingredient | Weight Percentage (of overall composition) |
|---|---|
| Amigel | 0.50 |

After the sixth phase is mixed thoroughly into the composition, the mixture continues to cool. Once the mixture cools to 50 °C, the seventh phase is added. The seventh phase comprises the ingredients in the following table:

| Name of Ingredient | Weight Percentage (of overall composition) |
|---|---|
| PVP-I | 7.50 |

Once the ingredients of the seventh phase are dissolved, the eighth phase is added, the eighth phase comprising the following ingredients:

| Name of Ingredient | Weight Percentage (of overall composition) |
|---|---|
| Sepigel | 2.00 |

### Example 3

An aqueous phase was first prepared by mixing the following components in the weight proportions listed. The aqueous phase was heated to 70 °C and mixed.

| Name of Ingredient | Weight Percentage (of overall composition) |
|---|---|
| Distilled Water, USP | 64.20 |
| Stearic Acid, NF | 2.50 |
| Benzalkonium Chloride | 0.10 |

Next the second phase was added, after which mixing and a temperature of 70 °C were maintained for 30 minutes.

| Name of Ingredient | Weight Percentage (of overall composition) |
|---|---|
| Cetyl Alcohol | 2.00 |

Next, the third phase was added. Each ingredient of the third phase, listed in the table below, was added in sequence while mixing and a temperature of 70 °C were maintained in the mixing vessel. The mixture was mixed for 10 minutes before the next addition commenced.

| Name of Ingredient | Weight Percentage (of overall composition) |
|---|---|
| Triton X-100 | 2.00 |
| Nonoxynol NP9 | 2.00 |
| Merquat 550 | 1.65 |
| Trolamine NF | 0.70 |
| Varisoft TA-100 | 1.70 |
| PVP-USP/NF | 0.40 |
| EDTA | 0.20 |
| Ajidew N100 | 0.10 |

Next, the fourth phase was added. Each ingredient in the table below was added in sequence while mixing an a temperature of 70 °C were maintained. The mixture was mixed for 10 minutes.

| Name of Ingredient | Weight Percentage (of overall composition) |
|---|---|
| Lauricidin | 4.25 |
| Beeswax | 0.50 |
| Floraester | 0.80 |

After 10 minutes of mixing, the second part of the fourth phase was added. Each of the ingredients in the table below were added in sequence. After the last ingredient was dissolved, heat was removed from the mixture.

| Name of Ingredient | Weight Percentage (of overall composition) |
|---|---|
| Jeechem GC | 1.00 |
| Arlacel 165 | 2.50 |

The fifth phase is then added. Each of the ingredients in the table below are added in sequence:

| Name of Ingredient | Weight Percentage (of overall composition) |
|---|---|
| Allantoin | 0.40 |
| Butylene Glycol | 4.00 |

The mixture is allowed to 58 °C after which the sixth phase is added. The sixth phase comprises the ingredients in the following table:

| Name of Ingredient | Weight Percentage (of overall composition) |
|---|---|
| Amigel | 0.50 |

After the sixth phase is mixed thoroughly into the composition, the mixture continues to cool. Once the mixture cools to 50 °C, the seventh phase is added. The seventh phase comprises the ingredients in the following table:

| Name of Ingredient | Weight Percentage (of overall composition) |
|---|---|
| PVP-I | 7.50 |

Once the ingredients of the seventh phase are dissolved, the eighth phase is added, the eighth phase comprising the following ingredients:

| Name of Ingredient | Weight Percentage (of overall composition) |
|---|---|
| Sepigel | 1.00 |

### Example 4

An aqueous phase was first prepared by mixing the following components in the weight proportions listed. The aqueous phase was heated to 70 °C and mixed.

| Name of Ingredient | Weight Percentage (of overall composition) |
|---|---|
| Distilled Water, USP | 63.60 |
| Stearic Acid, NF | 2.50 |
| Benzalkonium Chloride | 0.10 |

Next the second phase was added, after which mixing and a temperature of 70 °C were maintained for 30 minutes.

| Name of Ingredient | Weight Percentage (of overall composition) |
|---|---|
| Cetyl Alcohol | 2.40 |

Next, the third phase was added. Each ingredient of the third phase, listed in the table below, was added in sequence while mixing and a temperature of 70 °C were maintained in the mixing vessel. The mixture was mixed for 10 minutes before the next addition commenced.

| Name of Ingredient | Weight Percentage (of overall composition) |
|---|---|
| Triton X-100 | 2.00 |
| Nonoxynol NP9 | 2.00 |
| Merquat 550 | 1.65 |
| Trolamine NF | 0.70 |
| Varisoft TA-100 | 1.70 |
| PVP-USP/NF | 0.40 |
| EDTA | 0.20 |
| Ajidew N100 | 0.10 |

Next, the fourth phase was added. Each ingredient in the table below was added in sequence while mixing an a temperature of 70 °C were maintained. The mixture was mixed for 10 minutes.

| Name of Ingredient | Weight Percentage (of overall composition) |
|---|---|
| Lauricidin | 4.25 |
| Beeswax | 0.50 |
| Floraester | 1.00 |

After 10 minutes of mixing, the second part of the fourth phase was added. Each of the ingredients in the table below were added in sequence. After the last ingredient was dissolved, heat was removed from the mixture.

| Name of Ingredient | Weight Percentage (of overall composition) |
|---|---|
| Jeechem GC | 1.00 |
| Arlacel 165 | 2.50 |

The fifth phase is then added. Each of the ingredients in the table below are added in sequence:

| Name of Ingredient | Weight Percentage (of overall composition) |
|---|---|
| Allantoin | 0.40 |
| Butylene Glycol | 4.00 |

The mixture is allowed to 58 °C after which the sixth phase is added. The sixth phase comprises the ingredients in the following table:

| Name of Ingredient | Weight Percentage (of overall composition) |
|---|---|
| Amigel | 0.50 |

After the sixth phase is mixed thoroughly into the composition, the mixture continues to cool. Once the mixture cools to 50 °C, the seventh phase is added. The seventh phase comprises the ingredients in the following table:

| Name of Ingredient | Weight Percentage (of overall composition) |
|---|---|
| PVP-I | 7.50 |

Once the ingredients of the seventh phase are dissolved, the eighth phase is added, the eighth phase comprising the following ingredients:

| Name of Ingredient | Weight Percentage (of overall composition) |
|---|---|
| Sepigel | 1.00 |

### Example 5

An aqueous phase was first prepared by mixing the following components in the weight proportions listed. The aqueous phase was heated to 70 °C and mixed.

| Name of Ingredient | Weight Percentage (of overall composition) |
|---|---|
| Distilled Water, USP | 63.00 |
| Stearic Acid, NF | 2.75 |
| Benzalkonium Chloride | 0.10 |

Next the second phase was added, after which mixing and a temperature of 70 °C were maintained for 30 minutes.

| Name of Ingredient | Weight Percentage (of overall composition) |
|---|---|
| Cetyl Alcohol | 2.50 |

Next, the third phase was added. Each ingredient of the third phase, listed in the table below, was added in sequence while mixing and a temperature of 70 °C were maintained in the mixing vessel. The mixture was mixed for 10 minutes before the next addition commenced.

| Name of Ingredient | Weight Percentage (of overall composition) |
|---|---|
| Triton X-100 | 2.00 |
| Nonoxynol NP9 | 2.00 |
| Merquat 550 | 1.65 |
| Trolamine NF | 0.70 |
| Varisoft TA-100 | 1.70 |
| PVP-USP/NF | 0.40 |
| EDTA | 0.20 |
| Ajidew N100 | 0.10 |

Next, the fourth phase was added. Each ingredient in the table below was added in sequence while mixing an a temperature of 70 °C were maintained. The mixture was mixed for 10 minutes.

| Name of Ingredient | Weight Percentage (of overall composition) |
|---|---|
| Lauricidin | 4.25 |
| Beeswax | 0.50 |
| Floraester | 1.00 |

After 10 minutes of mixing, the second part of the fourth phase was added. Each of the ingredients in the table below were added in sequence. After the last ingredient was dissolved, heat was removed from the mixture.

| Name of Ingredient | Weight Percentage (of overall composition) |
|---|---|
| Jeechem GC | 1.00 |
| Arlacel 165 | 2.75 |

The fifth phase is then added. Each of the ingredients in the table below are added in sequence:

| Name of Ingredient | Weight Percentage (of overall composition) |
|---|---|
| Allantoin | 0.40 |
| Butylene Glycol | 4.00 |

The mixture is allowed to 58 °C after which the sixth phase is added. The sixth phase comprises the ingredients in the following table:

| Name of Ingredient | Weight Percentage (of overall composition) |
|---|---|
| Amigel | 0.50 |

After the sixth phase is mixed thoroughly into the composition, the mixture continues to cool. Once the mixture cools to 50 °C, the seventh phase is added. The seventh phase comprises the ingredients in the following table:

| Name of Ingredient | Weight Percentage (of overall composition) |
|---|---|
| PVP-I | 7.50 |

Once the ingredients of the seventh phase are dissolved, the eighth phase is added, the eighth phase comprising the following ingredients:

| Name of Ingredient | Weight Percentage (of overall composition) |
|---|---|
| Sepigel | 1.00 |

## Claims

1. A composition comprising:
4 weight percent to 12 weight percent of povidone-iodine complex,
0.1 weight percent to 2 weight percent of a tertiary amide, tertiary amide salt, and/or tertiary amide hydrate, 0.5 weight percent to 2 weight percent of low iodine reactive emollient, wherein the low iodine reactive emollient is selected from the group consisting of jojoba oil, hydrogenated jojoba esters, hydrogenated polybutenes, isododecane, glyceryl tribehenate, hydrogentated castor oil, palm oil, and combinations thereof; at least one surfactant; and
water;
wherein the composition has a pH ranging from 2.0 to 3.5.

2. The composition of claim 1, wherein the weight percent of the povidone-iodine complex is between 5 and 10.

3. The composition of claim 1, wherein the weight percent of the povidone-iodine complex is between 6 and 9.

4. The composition of claim 1, wherein the low iodine reactive emollient comprises hydrogenated jojoba esters.

5. The composition of claim 1, wherein the surfactant is selected from the group consisting of a polyethylene glycol of 4-(1,1,3,3-tetramethybutyl)-phenyl, glyceryl stearate, PEG-100 stearate, laureth-12, Non-oxynol-9 or combinations thereof.

6. The composition of claim 1, wherein the tertiary amide, tertiary amide hydrate, tertiary amide salt has the formula: 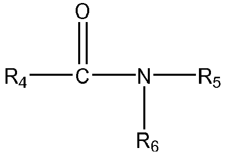
wherein R₄ is a saturated fatty group containing 11-60 carbon atoms;
R₅ and R₆ are independently aryl, aryl C₁₋₆ alkyl, a saturated fatty group containing 11-29 carbon atoms, or R₇; and
R₇ is
R₁-Ar-O-R₂-O-R₃
wherein R₂ and R₃ are alkyl groups containing 1-6 carbon atoms and R₁ is an alkyl group containing 1-15 carbon atoms and Ar is aryl.

7. The composition of claim 6, wherein R₄ is a saturated fatty group containing 15-46 carbon atoms

8. The composition of claim 6, wherein Ar is a phenyl group.

9. The composition of claim 6, wherein R₅ is a benzyl group.

10. The composition of claim 6, wherein the tertiary amide is benzethonium stearamide or benzalkonium stearamide.

11. The composition of claim 1, wherein the composition is in the form of a paste, cream or ointment.

## Patentansprüche

1. Zusammensetzung, umfassend:
4 Gewichtsprozent bis 12 Gewichtsprozent Povidon-Iod-Komplex,
0,1 Gewichtsprozent bis 2 Gewichtsprozent eines tertiären Amids, tertiären Amidsalzes und/oder eines tertiären Amidhydrats, 0,5 Gewichtsprozent bis 2 Gewichtsprozent eines Weichmachers, der schwach mit Iod reagiert, wobei der Weichmacher aus der Gruppe ausgewählt wird, bestehend aus Jojobaöl, hydrierten Jojoba-Estern, hydrierten Polybutenen, Isododecan, Glyceryltribehenat, hydriertem Rizinusöl, Palmöl und Kombinationen davon; mindestens einem Tensid; und Wasser;
wobei die Zusammensetzung einen pH-Wert hat, der in einem Bereich von 2,0 bis 3,5 ist.

2. Die Zusammensetzung nach Anspruch 1, wobei der Gewichtsprozentsatz des Povidon-Iod-Komplexes zwischen 5 und 10 ist.

3. Die Zusammensetzung nach Anspruch 1, wobei der Gewichtsprozentsatz des Povidon-Iod-Komplexes zwischen 6 und 9 ist.

4. Die Zusammensetzung nach Anspruch 1, wobei der Weichmacher, der schwach mit Iod reagiert, hydrierte Jojoba-Ester umfasst.

5. Die Zusammensetzung nach Anspruch 1, wobei das Tensid aus der Gruppe ausgewählt wird, bestehend aus einem Polyethylenglykol von 4-(1,1,3,3-Tetramethylbutyl)-phenyl, Glycerylstearat, PEG-100-Stearat, Laureth-12, Nonoxynol-9 oder Kombinationen davon.

6. Die Zusammensetzung nach Anspruch 1, wobei das tertiäre Amid, tertiäre Amidhydrat, tertiäre Amidsalz, die Formel hat: 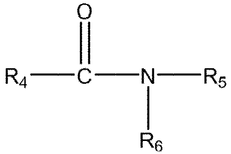
wobei R₄ eine gesättigte Fettsäurengruppe ist, enthaltend 11 bis 60 Kohlenstoffatome;
R₅ und R₆ unabhängig Aryl, Aryl C₁₋₆ Alkyl, eine gesättigte Fettsäurengruppe, enthaltend 11 bis 29 Kohlenstoffatome, oder R₇ sind; und
R₇ ist
R₁-Ar-O-R₂-O-R₃
wobei R₂ und R₃ Alkylgruppen sind, enthaltend 1 bis 6 Kohlenstoffatome, und R₁ eine Alkylgruppe ist, enthaltend 1 bis 15 Kohlenstoffatome, und Ar Aryl ist.

7. Die Zusammensetzung nach Anspruch 6, wobei R₄ eine gesättigte Fettsäurengruppe ist, enthaltend 15 bis 46 Kohlenstoffatome.

8. Die Zusammensetzung nach Anspruch 6, wobei Ar eine Phenylgruppe ist.

9. Die Zusammensetzung nach Anspruch 6, wobei R₅ eine Benzylgruppe ist.

10. Die Zusammensetzung nach Anspruch 6, wobei das tertiäre Amid Benzethonium-Stearamid oder Benzalkonium-Stearamid ist.

11. Die Zusammensetzung nach Anspruch 1, wobei die Zusammensetzung in der Form einer Paste, Creme oder Salbe ist.

## Revendications

1. Une composition comprenant:
de 4 pour cent en poids à 12 pour cent en poids d'un complexe de polyvidone iodée,
de 0,1 pour cent en poids à 2 pour cent en poids d'un amide tertiaire, d'un sel d'amide tertiaire, et/ou d'un hydrate d'amide tertiaire,
de 0,5 à 2 pour cent en poids d'un émollient réactif à faible teneur en iode, l'émollient réactif à faible teneur en iode étant choisi dans le groupe constitué par l'huile de jojoba, des esters de jojoba hydrogénés, des polybutènes hydrogénés, de l'isododécane, du tribéhénate de glycéryle, de l'huile de ricin hydrogénée, de l'huile de palme et des combinaisons de ceux-ci ; au moins un tensioactif ; et
de l'eau ;
dans laquelle la composition présente un pH compris entre 2,0 et 3,5.

2. La composition selon la revendication 1, dans laquelle le pourcentage en poids du complexe de polyvidone iodée est compris entre 5 et 10.

3. La composition selon la revendication 1, dans laquelle le pourcentage en poids du complexe de polyvidone iodée est compris entre 6 et 9.

4. La composition selon la revendication 1, dans laquelle l'émollient réactif à faible teneur en iode comprend des esters de jojoba hydrogénés.

5. La composition selon la revendication 1, dans laquelle le tensioactif est choisi dans le groupe constitué d'un polyéthylène glycol de 4-(1,1,3,3-tétraméthylbutyle)-phényle, de stéarate de glycéryle, de PEG-100 stéarate, de laureth-12, de nonoxynol-9 ou des combinaisons de ceux-ci.

6. La composition selon la revendication 1, dans laquelle l'amide tertiaire, l'hydrate d'amide tertiaire et le sel d'amide tertiaire ont la formule : 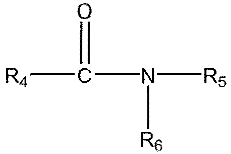
dans laquelle R₄ est un groupe gras saturé contenant 11 à 60 atomes de carbone;
R₅ et R₆ sont indépendamment un aryle, un aryle en C₁₋₆ alkyle, un groupe gras saturé contenant 11 à 29 atomes de carbone, ou R₇; et
R₇ est
R₁-Ar-O-R₂-O-R₃
dans laquelle R₂ et R₃ sont des groupes alkyle contenant de 1 à 6 atomes de carbone et R₁ est un groupe alkyle contenant de 1 à 15 atomes de carbone, et Ar est un aryle.

7. La composition selon la revendication 6, dans laquelle R₄ est un groupe gras saturé contenant de 15 à 46 atomes de carbone.

8. La composition selon la revendication 6, dans laquelle Ar est un groupe phényle.

9. La composition selon la revendication 6, dans laquelle R₅ est un groupe benzyle.

10. La composition selon la revendication 6, dans laquelle l'amide tertiaire est un stéaramide de benzonium ou un stéaramide de benzalkonium.

11. La composition selon la revendication 1, la composition se présentant sous la forme d'une pâte, d'une crème ou d'un onguent.
